# EUROPEAN PATENT SPECIFICATION

(11) **EP 2 736 909 B1**
(45) Date of publication and mention of the grant of the patent: **29.03.2017**
(21) Application number: 12740505.8
(22) Date of filing: 27.07.2012
(51) Int. Cl.: C07D 487/04

(54) **PROCESS FOR THE PREPARATION OF SITAGLIPTIN AND ITS PHARMACEUTICALLY ACCEPTABLE SALTS**
VERFAHREN ZUR HERSTELLUNG VON SITAGLIPTIN UND DESSEN PHARMAZEUTISCH VERTRÄGLICHEN SALZEN
PROCÉDÉ DE PRÉPARATION DE SITAGLIPTIN ET SES SELS PHARMACEUTIQUEMENT ACCEPTABLES

(30) Priority: 27.07.2011 SI 201100277
(43) Date of publication of application: 04.06.2014
(73) Proprietor: Farma GRS, d.o.o., 8000 Novo mesto (SI)
(72) Inventor: ZUPANCIC, Silvo, 8000 Novo mesto (SI); ZUPET, Rok, 1211 Ljublijana (SI)
(74) Representative: Andrae | Westendorp Patentanwälte Partnerschaft
(86) International application number: PCT/EP2012/003214
(87) International publication number: WO 2013/013833

(56) References cited:
- WO-A1-03/004498
- WO-A1-2010/131025
- "A process for preparing (2R)-4-oxo-4-[3-(trifluoromethyl)-5,6-dihy dro[1,2,4]triazolo[4,3-a]pyrazin-7(8H)-yl] -1-(2,4,5-trifluorophenyl)butan-2-amine", IP.COM JOURNAL, IP.COM INC., WEST HENRIETTA, NY, US, 27 May 2010 (2010-05-27), XP013138386, ISSN: 1533-0001 cited in the application
- K.B. HANSEN ET AL: ORGANIC PROCESS RESEARCH & DEVELOPMENT, vol. 9, no. 5, 2005, pages 634-639, XP002682826, cited in the application

## Description

### FIELD OF THE INVENTION

The present invention belongs to the field of organic chemistry. More particularly, the present invention describes an improved process for the preparation of sitagliptin and its pharmaceutically acceptable salts. The process is cost effective, environmentally friendly, inexpensive and easily scaled up to commercial level.

### BACKGROUND OF THE INVENTION

Sitagliptin, chemicaly named as (R)-4-oxo-4-[3-(trifluoromethyl)-5,6-dihydro[1,2,4]triazolo[4,3-*a*]pyrazin-7(8*H*)-yl]-1-(2,4,5-trifluorophenyl)butan-2-amine, has the following chemical structure:

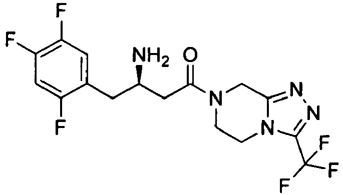

Sitagliptin is an oral antihyperglycemic (antidiabetic) drug of the dipeptidyl peptidase-4 (DPP-4) inhibitor class. It is marketed as sitagliptin phosphate by Merck Co. under the trade name Januvia™. This enzyme-inhibiting drug is used either alone or in combination with other oral antihyperglycemic agents (such as for example metformin or a thiazolidinedione) for treatment of diabetes mellitus type 2.

Sitagliptin works to competitively inhibit the enzyme dipeptidyl peptidase 4 (DPP-4). This enzyme breaks down the incretins GLP-1 and GIP, gastrointestinal hormones released in response to a meal. By preventing GLP-1 and GIP inactivation, they are able to potentiate the secretion of insulin and suppress the release of glucagon by the pancreas. This drives blood glucose levels towards normal. As the blood glucose level approaches normal, the amounts of insulin released and glucagon suppressed diminishes, thus tending to prevent an "overshoot" and subsequent low blood sugar (hypoglycemia) which is seen with some other oral hypoglycemic agents.

The substance was first described in WO 2003/004498 A1 wherein the general synthesis of sitagliptin by coupling of the beta-amino acid fragment with heterocyclic fragment as shown in scheme 1 is disclosed.

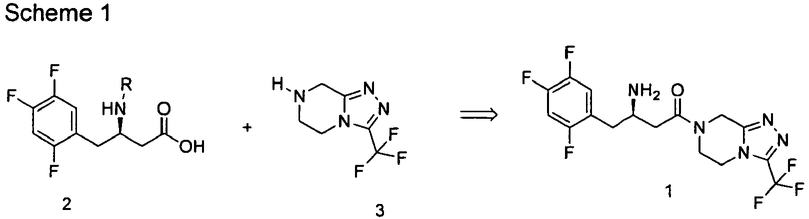

This reaction is carried out under standard peptide coupling conditions, using 1-ethyl-3-3(dimethylaminopropyl)carbodiimide (EDC), 1-hydroxybenzotriazole (HOBT) and a base usually diisopropylethylamine, in a solvent such as *N,N*-dimethylformamide (DMF) or dichloromethane (DCM). The protecting group is then removed with trifluoroacetic acid or methanolic hydrogen chloride in the case of *tert*-butoxycarbonyl as protecting group. The disadvantage of the process is rather low yield obtained after chromatographic purification and the use of reagents such as HOBT which is reported to be explosive therefore its use should be omitted.

A crystalline dihydrogenphosphate salt of sitagliptin phosphate is described in WO 2005/003135.

The synthesis of sitagliptin by employing stereoselective reduction is described for example in WO 2004/085378, WO 2004/085661, WO 2004/085378.

In Organic Process Research & Development 2005, 9, 634-639 a process is disclosed where compound of formula 2, wherein R is OBn (Bn denotes benzyl group) is coupled with compound of formula 3 in the presence of *N*-methylmorpholine (NMM) as a base and EDC-HCl as a coupling agent affording desired compound in more than 99% assay yield. The drawback of the process resides in benzyl protecting group which requires catalytical hydrogenation with 10% Pd on carbon for its removal.

IPCOM000196065D discloses a process for the production of sitagliptin wherein (R)-3-((*tert*-butoxycarbonyl)amino)-4-(2,4,5-trifluorophenyl)butanoic acid or its salt is reacted with 3-(trifluoromethyl)-5,6,7,8-tetrahydro-[1,2,4]triazolo[4,3-a] pyrazine or its salt in the presence of triethylamine as an organic base and propyl phosphonic anhydride as a coupling agent or *N*-methylmorfoline as an organic base and 1-ethyl-3-(3-dimethylaminopropyl)carbodiimide (EDC) or *N,N'*-dicyclohexylcarbodiimide (DCC) as a coupling agent in an organic solvent which is ethyl acetate. The final compound is isolated as oil which can be directly transformed into a salt or isolated in a solid form in further steps. The reported HPLC purity is 95.33% and it is reported that the obtained oil was observed to change its consistency over time towards wax.

In J. Med. Chem. 2005, 48, 141-151 condensation in the presence of EDC and HOBT in DMF as a solvent is reported. The drawback of the process still remained in rather low yield of only 71% and in purification by flash chromatography which is not suitable on industrial scale production.

Journal of Chemical Research 2010, 230-232 describes the condensation reaction in the presence of EDC-HCl and HOBT in anhydrous DCM and Et₃N. The obtained product is recrystallized from toluene. The deprotection step is performed with conc. HCl and methanol. In addition, the condensation of compound of formula 2 with the compound of formula 3 in the presence of EDC or EDC-HCl and/or in the presence of HOBT and in the presence of an organic base in an organic solvent and further isolation of the intermediate product before performing deprotection step is disclosed in several other literature such as for example in WO2009064476, WO2010131025.

However, there still exists a need for an efficient and economical synthesis of sitagliptin or its pharmaceutically acceptable salts, which provides highly pure final product with high yields.

### SUMMARY OF THE INVENTION

According to the first embodiment, the present invention provides a process, preferably a one pot process for the preparation of sitagliptin or its pharmaceutically acceptable salts of formula 1 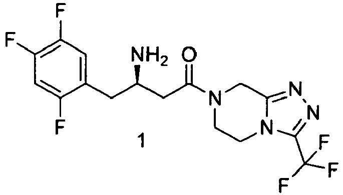 comprising the steps of
a) condensation of compound of formula 2 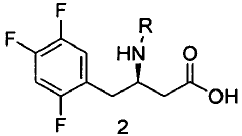 wherein R is amino protecting group which can be removed, preferably under acidic condition, that can be selected from the group consisting of, but not limited to, carbamate group, *tert*-Butyloxycarbonyl (BOC), Carbobenzyloxy (Cbz) group, *p-*Methoxybenzyl carbonyl (Moz or MeOZ) group, 9-Fluorenylmethyloxycarbonyl (FMOC) group, Acetyl (Ac) group, Benzoyl (Bz) group, Benzyl (Bn) group, *p*-Methoxybenzyl (PMB), 3,4-Dimethoxybenzyl (DMPM); preferably carbamate group or BOC, with compound of formula 3 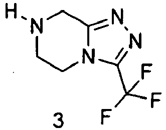 which may be used in its free base form or in a form of a salt, particularly in the form of hydrochloride salt, in the presence of a coupling agent that can be selected from the group consisting of, but not limited to, phosgene, diphosgene, triphosgene, carbonildiimidazole, chlorothionyl imidazole, thionyl diimidazole, dicyclohexylcarbodiimide (DCC), of *N*-(3-Dimethylaminopropyl)-*N'*-ethylcarbodiimide hydrochloride-a (EDC-HCl), tetramethyluronium salts such as *O*-(benzotriazol-1-yl)-*N,N, N',N'*-tetramethyluronium hexafluorophosphate (HBTU), *O*-(benzotriazol-1-yl)-*N,N,N', N'*-tetramethyluronium tetrafluoroborate (TBTU), or *O*-(7-aza-benzotriazol-1-yl)-*N,N,N', N'*-tetramethyluronium hexafluorophosphate, (HATU), preferably *N*-(3-Dimethylaminopropyl)-*N'*-ethylcarbodiimide hydrochloride-a (EDC-HCl) and a basic solvent that is *N*-methylimidazole to obtain the compound of formula 4 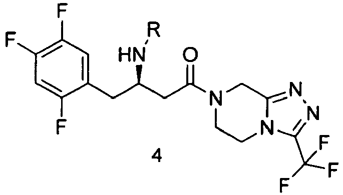 and
b) deprotection of compound of formula 4 to obtain sitagliptin base that can be further transformed into its pharmaceutically acceptable salt.

According to another embodiment the HPLC purity of the obtained sitagliptin can be from 99 to 100%, preferably from 99.5 to 100%, more preferably from 99.7 to 100%.

According to another embodiment the percent enantiomeric excess of sitagliptin prepared by the process according to the present invention is more than 99.5%, preferably more than 99,8%, more preferably more than 99,9% (by HPLC).

According to another embodiment the obtained sitagliptin is converted into one of its pharmaceutically acceptable salts prepared from any inorganic or organic base or acid, such as for example hydrochloride, phosphate, dihydrogenphosphate, tartrate, benzensulfonate, sulfate, acetate, preferably hydrochloride, phosphate and dihydrogenphosphate.

According to another embodiment the present invention relates to the pharmaceutical composition comprising sitagliptin or its pharmaceutically acceptable salts prepared by the process according to the present invention and optionally at least one other active substance and at least one pharmaceutically acceptable excipient.

According to another embodiment the present invention relates to sitagliptin or its pharmaceutically acceptable salts prepared by the process according to the present invention for use in the treatment, controlling or preventing diabetes, non-insulin dependent (Type 2) diabetes mellitus, hyperglycemia, obesity, insulin resistance, one or more lipid disorders selected from the group consisting of, but not limited to, dyslipidemia, hyperlipidemia, hypertriglyceridemia, hypercholesterolemia, low HDL, and high IDL, atherosclerosis, one or more disorders selected from the group consisting of, but not limited to, neutropenia, neuronal disorders, tumor metastasis, benign prostatic hypertrophy, gingivitis, hypertension and osteoporosis, or one or more conditions selected from, but not limited to, intestinal injury, inflammatory bowel disease, Crohn's disease, and ulcerative colitis.

### DETAILED DESCRIPTION OF THE INVENTION

The authors of the present invention surprisingly found out that the usage of *N-*methylimidazole as a basic solvent in the condensation reaction of compound of formula 2 with 3-(trifluoromethyl)-5,6,7,8-tetrahydro-[1,2,4]triazolo[4,3-a] pyrazine or its hydrochloride salt and the usage of the same solvent that is *N*-methylimidazole in the subsequent deprotection reaction enables one pot synthesis of sitagliptin and its pharmaceutically acceptable salts.

Moreover, the process according to the present invention provides sitagliptin and its pharmaceutically acceptable salts with high chemical and enantiomerical purity in a cost effective, environmentally friendly and easily scale up manner.

According to the first embodiment, the present invention provides a process, preferably a one pot process for the preparation of sitagliptin or its pharmaceutically acceptable salts of formula 1 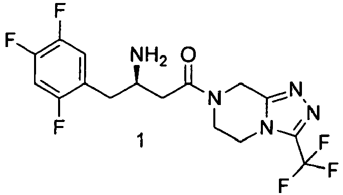 comprising the steps of
a) condensation of compound of formula 2 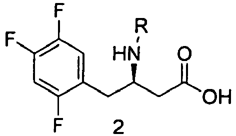 wherein R is amino protecting group which can be removed under acidic condition with compound of formula 3 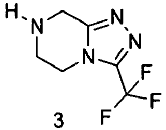 which may be used in its free base form or in a form of a salt, particularly in the form of hydrochloride salt, in the presence of a coupling agent and a basic solvent that is *N-*methylimidazole to obtain the compound of formula 4 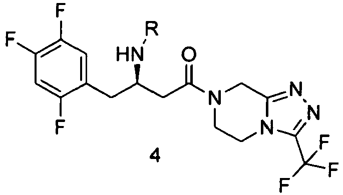 and
b) deprotection of compound of formula 4 to obtain sitagliptin base that can be further transformed into its pharmaceutically acceptable salt.

Amino protecting group which can be removed under acidic condition can be selected from the group consisting of, but not limited to, carbamate group, *tert*-Butyloxycarbonyl (BOC), Carbobenzyloxy (Cbz) group, *p*-Methoxybenzyl carbonyl (Moz or MeOZ) group, 9-Fluorenylmethyloxycarbonyl (FMOC) group, Acetyl (Ac) group, Benzoyl (Bz) group, Benzyl (Bn) group, *p*-Methoxybenzyl (PMB), 3,4-Dimethoxybenzyl (DMPM); preferably groups which can be removed by treatment with an acid such as carbamate group, BOC.

The coupling agent used in the process according to the present invention can be selected from the group consisting of, but not limited to, phosgene, diphosgene, triphosgene, carbonildiimidazole, chlorothionyl imidazole, thioniyl diimidazole, dicyclohexylcarbodiimide (DCC), of *N*-(3-Dimethylaminopropyl)-*N'*-ethylcarbodiimide hydrochloride-a (EDC-HCl), tetramethyluronium salts such as *O*-(benzotriazol-1-yl)-*N,N, N',N'*-tetramethyluronium hexafluorophosphate (HBTU), *O-*(benzotriazol-1-yl)*-N,N,N', N'*-tetramethyluronium tetrafluoroborate (TBTU), or *O*-(7-aza-benzotriazol-1-yl)-*N,N,N', N'*-tetramethyluronium hexafluorophosphate, (HATU), preferably *N*-(3-Dimethylaminopropyl)-*N'*-ethylcarbodiimide hydrochloride-a (EDC-HCl) is used.

The advantages of using *N*-methylimidazole as an organic solvent in the conversion step of the process according to the present invention are the following:
- excellent conversion of starting compounds which means that at least 95% of starting compound 2 and of starting compound 3 is converted to the compound of formula 4,
- compound of formula 3 can be used either in the form of a base or in the form of an acidic salt, which means that the addition of an additional base to the reaction mixture is no needed,
- due to good solubility of *N*-methylimidazole in water isolation by extraction is easy to handle,
- there is no need to use any additional compound for avoiding racemisation, as for example, but not limited to, HOBT,
- the purification of the obtained product by column chromatography is not needed,
- because of good solubility of starting material in *N*-methylimidazole the amount of spent solvent can be lower than in case when using other solvents known from the state of the art, this means that the reaction can be performed at high concentrations of reagents,
- *N*-methylimidazole is not environmentally hazardous and can be regenerated.

The starting compound of formula 2 can be prepared by any known process disclosed in the prior art, such as for example, but not limited to, IPCOM000176671, Bioorganic and Medicinal Chemistry Letters; vol. 17; nb. 9; (2007); p. 2622 - 2628, Journal of Medicinal Chemistry; vol. 48; nb. 1; (2005); p. 141 - 151, Journal of Chemical Research, Synopses; nb. 4; (2010); p. 230 - 232, European Journal of Medicinal Chemistry; vol. 45; nb. 11; (2010); p. 4953 - 4962, Tetrahedron: Asymmetry; vol. 17; nb. 2; (2006); p. 205 - 209, EP2308829, EP2230241, WO2010131025, WO201012278, WO201078440, WO200623750, WO20047468, WO200382817, US2008280913.

The starting compound of formula 3 can be prepared by any known process disclosed in the prior art, such as for example, but not limited to, IPCOM000176303D, Organic Letters, vol. 7; nb. 6; (2005); p. 1039 -1042, Journal of Medicinal Chemistry; vol. 48; nb. 1; (2005); p. 141 - 151, Journal of Medicinal Chemistry; vol. 48; nb. 1; (2005); p. 141 - 151, EP2270009, WO200985990, WO200750485, WO200735198, WO2006119260, WO200681151, WO200633848, WO200623750, WO20069886, WO200597733, WO200485378, WO200458266, US2010331541, US2008280913.

In a preferred embodiment the HPLC purity of starting compound 2 and starting compound 3 can be at least 95%, preferably at least 96%, more preferably at least 97.5%.

In a preferred embodiment the percent enantiomeric excess of the compound of formula 2 can be more than 98.5% (by HPLC).

In a preferred embodiment the condensation reaction of the compound of formula 2 with the compound of formula 3 is conducted at a temperature between 0°C and 60°C, preferably between 10°C and 30°C.

In a further preferred embodiment the condensation reaction of the compound of formula 2 with the compound of formula 3 is carried out between 2 and 24 hours, preferably between 3 and 8 hours.

Ratio between any reagents used in the process according to the present invention is equimolar. In addition, the concentration of the compound of formula 3 in reaction solvent can be between 50g/L and 600g/L, preferably between 100g/L ang 500g/L.

In a preferred embodiment the deprotection of in-situ formed compound of formula 4 is performed in an acid, preferably HCl. HCl can be used as an alcoholic solution or concentrated aqueous solution. Preferably concentrated aqueous solution of HCl is used.

In a preferred embodiment the deprotection of in-situ formed compound of formula 4 is conducted at a temperature between 0°C and 60°C, preferably between 10°C and 30°C.

In a further preferred embodiment the deprotection of in-situ formed compound of formula 4 is carried out between 2 and 24 hours, preferably between 3 and 10 hours.

The obtained sitagliptin can be isolated by any suitable method from the state of the art, as for example, but not limited to, extraction, evaporation, filtration, recrystallisation, distillation, chromatography and similar.

According to the further embodiment the HPLC purity of the obtained sitagliptin can be from 99 to 100%, preferably from 99.5 to 100%, more preferably from 99.7 to 100%.

According to the further embodiment the percent enantiomeric excess of sitagliptin prepared by the process according to the present invention is more than 99.5%, preferably more than 99,8%, more preferably more than 99,9% (by HPLC).

In a preferred embodiment the overall yield of the process for the production of sitagliptin according to the present invention can be at least 75%, preferably at least 80 more preferably at least 85%.

In another embodiment the obtained sitagliptin is converted into one of its pharmaceutically acceptable salts prepared from any inorganic or organic base or acid, such as for example salts described in, but not limited to, WO2003004498, WO2005003135, WO2005020920, WO2005030127, WO2005072530, WO2006033848, WO2006104997, WO2007035198, KR20070111099, WO2009084024, WO2009070314, WO2009085990, WO2009120746, US2009247532, WO2010000469, WO2010012781, WO2010032264, WO2010092090, US2010249140, WO2010122578, WO2010131035, WO2011018494, preferably the salt can be hydrochloride, phosphate, dihydrogenphosphate, tartrate, benzensulfonate, sulfate, acetate, more preferably hydrochloride, phosphate, dihydrogenphosphate. The conversion can be conducted according to any known processes such as processes described in the above mentioned references.

In a preferred embodiment the obtained sitagliptin or its pharmaceutically acceptable salts can be in the form of the amorphous substance or crystalline (polymorphic) substance. Moreover, it is contemplated in the context of the present invention to prepare any pharmaceutically acceptable hydrate form of sitagliptin and its pharmaceutically acceptable salts.

In a still another embodiment the sitagliptin base obtained according to the process of the present invention is converted into hydrochloride by mixing sitagliptin base with HCl. The obtained sitagliptin HCl salt can be isolated or directly in-situ used in the process for the preparing pharmaceutical composition. The obtained sitagliptin HCl salt can be in an amorphous form such as for example described in, but not limited to, SI P-201100115, or in a crystalline form such as for example described in, but not limited to, WO2005072530, KR20070111099, WO2010000469.

In a preferred embodiment the average particle size of the obtained sitagliptin or its pharmaceutically acceptable salts can be from 0.1 to 600 µm, preferably from 1 to 250 µm and more preferably from 10 to 50 µm. The term "average particle size" as used herein refers to the volume mean diameter of the particles. The volume mean diameter was determined by laser light scattering using a Malvern-Mastersizer Apparatus MS 2000 equiped with Hydro S dispersion unit. Vegetable oil was used as the dilution medium.

In another embodiment the present invention relates to pharmaceutical composition comprising sitagliptin or its pharmaceutically acceptable salts prepared by the process according to the present invention and other pharmaceutically acceptable excipients. Optionally, other active substances can be present in the pharmaceutical composition according to the present invention and can be administered either separately or in the same pharmaceutical composition. The other active substances can be selected from, but not limited to, other dipeptidyl peptidase IV inhibitors, biguanides, fenofibric acid derivatives, sulfonylureas, glitazones and any mixtures thereof.

The pharmaceutical composition according to the present invention can be administered by oral, parenteral, by inhalation, nasal, vaginal, rectal, sublingual, or topical in suitable dosage unit form comprising pharmaceutically acceptable excipients appropriate for each route of administration. The pharmaceutically acceptable excipients can be selected from, but not limited to, fillers, binders, disintegrants, lubricants, colouring agents, adsorbents, surfactants, film formers, plastizers and the like.

Preferably the pharmaceutical composition according to the present invention comprises from 1 mg to 200 mg, preferably from 10 to 150 mg, more preferably from 25 to 100 mg of sitagliptin or its pharmaceutically acceptable salts.

In another embodiment the present invention relates to sitagliptin or its pharmaceutically acceptable salts prepared by the process according to the present invention for use in the treatment, controlling or preventing diabetes, non-insulin dependent (Type 2) diabetes mellitus, hyperglycemia, obesity, insulin resistance, one or more lipid disorders selected from the group consisting of, but not limited to, dyslipidemia, hyperlipidemia, hypertriglyceridemia, hypercholesterolemia, low HDL, and high IDL, atherosclerosis, one or more disorders selected from the group consisting of, but not limited to, neutropenia, neuronal disorders, tumor metastasis, benign prostatic hypertrophy, gingivitis, hypertension and osteoporosis, or one or more conditions selected from, but not limited to, intestinal injury, inflammatory bowel disease, Crohn's disease, and ulcerative colitis.

We have surprisingly found out that one pot process to the production of sitagliptin according to the present invention from (R)-3-((tert-butoxycarbonyl)amino)-4-(2,4,5-trifluorophenyl)butanoic acid that takes place in *N*-methylimidazole as an organic solvent results in practically complete conversion of reactants therefore providing highly pure final product almost without any by-products. In addition, the process according to the present invention is cost effective, environmentally friendly and easily scaled up.

The present invention is illustrated by the following Examples without being limited thereto. With the examples we demonstrate the advantage of the process according to the present invention.

### EXAMPLES

### Reference example 1

### a) Condensation step

The mixture of 4.02 g of Boc(R)-3-Amino-(2,4,5-trifluorophenyl)butanoic acid, 2.77 g of 3-(Trifluoromethyl)-5,6,7,8-tetrahydro-[1,2,4]triazolo[4,3-a] pyrazine hydrochloride-a and 25 ml of pyridine is cooled to 0°C. Then 2.73 g of N-(3-Dimethylaminopropyl)-*N'-*ethylcarbodiimide hydrochloride-a (EDC) is added and the mixture is stirred for 16h at room temperature. The to the reaction mixture 100 ml of isopropyl acetate and 100ml of water are added. The phases are separated and aqueous phase is again extracted with 100 ml of ethyl acetate. Collected organic phases are washed with 100 ml of water and then to organic phase 100 ml of water is added and pH of the mixture is adjusted to around 3. Phases are separated and organic phase is evaporated. The residue is collected and dried.
Yield: 5.88g (95%)
HPLC: 97.6 %

### b) Deprotection step

The mixture of 5.4 g of (R)-*tert*-butyl (4-oxo-4-(3-(trifluoromethyl)-5,6-dihydro-[1,2,4]triazolo[4,3-a]pyrazin-7(8H)-yl)-1-(2,4,5-trifluorophenyl)butan-2-yl)carbamate and 27 ml of solution of 1.25M HCl in ethanol is stirred for 16h at room temperature. After the reaction is completed the mixture is evaporated and 37 ml of water is added and pH of the mixture is adjusted with 1 M NaOH to 10-12. Then 37 ml of isopropyl acetate is added, stirred and phases are separated. Aqueous phase is reextracted with 37 ml of isopropyl acetate. Collected organic phases are washed with 37 ml of water and evaporated. To the residue 27 ml of TBME is added. The obtained suspension is cooled below 0°C and then filtered and dried.
Yield for the deprotection step: 90%
Yield for the whole process: 85%
HPLC: 99.0%, *R* isomer 100.0% S isomer 0.0%

**HPLC method for chromatographic purity**

| | |
|---|---|
| **Column:** | Ascentis Express Phenyl-Hexyl, (100 x 4.6)mm, 2.7µm particles, |
| **Eluent A:** | 0.1% triethylamine pH=7.0 |
| **Eluent B:** | acetonitrile |

**Gradient:**

| Time (min) | Eluent A (%) | Eluent B (%) |
|---|---|---|
| 0 | 90 | 10 |
| 7 | 90 | 10 |
| 25 | 60 | 40 |
| 34 | 20 | 80 |
| 37 | 20 | 80 |
| 40 | 90 | 10 |

| | |
|---|---|
| **Post time:** | 4 min |
| **Flow-rate:** | 0.8 ml/min |
| **Detection:** | UV, wavelength 210 nm |
| **Injection volume:** | 2 µl |
| **Column temperature:** | 25°C |
| **Diluent:** | acetonitrile : water (50 : 50) |

**HPLC method for enantiomeric purity**

| | |
|---|---|
| **Column:** | Chiralpak AD-H, (250 x 4.6)mm, 5 µm particles, |
| **Mobile phase:** | ethanol : hexane : EDA in the ratio 600:400:1 (V/V/V) |
| **Flow-rate:** | 1.0 ml/min |
| **Detection:** | UV, wavelength 210 nm |
| **Injection volume:** | 20 µl |
| **Column temperature:** | 25°C |
| **Diluent:** | methanol |

### Example 1

The mixture of 8.04 g of (*R*)-3-((*tert*-butoxycarbonyl)amino)-4-(2,4,5-trifluorophenyl)butanoic acid (HPLC: 99.9%, ee (by HPLC): 99.9%), 5.54 g of 3-(trifluoromethyl)-5,6,7,8-tetrahydro-[1,2,4]triazolo[4,3-a]pyrazine hydrochloride (HPLC: 99.6%) and 40 ml of *N*-methylimidazole is cooled to 0°C. Then 5.46 g of *N*-(3-Dimethylaminopropyl)-*N'*-ethylcarbodiimide hydrochloride-a (EDC-HCl) is added and the mixture is stirred for 16 h at room temperature. To the reaction mixture 200 ml of isopropyl acetate and 200 ml of water are added. The phases are separated and aqueous phase is again extracted with 100 ml of ethyl acetate. Collected organic phases are washed with 200 ml of water (2×). The organic phase is evaporated to around 100 ml.

To concentrated organic phase 20 ml of 8M HCl/EtOH is added and the mixture is stirred at room temperature for 18 h. After the reaction is completed 50 ml of water is added and pH of the mixture is adjusted with 1M NaOH to 10-12. Then phases are separated. Aqueous phase is reextracted with 100 ml of isopropyl acetate. Collected organic phases are washed with 80 ml of water (2×) and evaporated. To the residue 60 ml of tert-butyl methyl ether is added. The obtained suspension is cooled below 0°C and then filtered and dried.
Yield: 8.84g (90%)
HPLC: 99.86%, R isomer 100.0% S isomer 0.0%

### Example 2

The mixture of 4.02 g of (*R*)-3-((*tert*-butoxycarbonyl)amino)-4-(2,4,5-trifluorophenyl)butanoic acid, 2.77 g of 3-(trifluoromethyl)-5,6,7,8-tetrahydro-[1,2,4]triazolo[4,3-a]pyrazine hydrochloride and 20 ml of *N*-methylimidazole is cooled to 0°C. Then 2.73 g of *N*-(3-Dimethylaminopropyl)-*N'*-ethylcarbodiimide hydrochloride-a (EDC) is added and the mixture is stirred for 6 h at room temperature. To the reaction mixture 100 ml of isopropyl acetate and 100 ml of water are added. The phases are separated and aqueous phase is again extracted with 50 ml of ethyl acetate. Collected organic phases are washed with 100 ml of water (2×). The organic phase is evaporated to around 45 ml.

To concentrated organic phase 7.5 ml of conc. HCl is added and the mixture is stirred at room temperature for 18 h. After the reaction is completed 25 ml of water is added and pH of the mixture is adjusted with aq. sol. Of NaOH to 10-12. Then phases are separated. Aqueous phase is reextracted with 500 ml of isopropyl acetate. Collected organic phases are washed with 40 ml of water (2×) and evaporated. To the residue 60 ml of tert-butyl methyl ether is added. The obtained suspension is cooled below 0°C and then filtered and dried.
Yield: 4.26 (86%)
HPLC: 99.84%, R isomer 100.0% S isomer 0.0%

## Claims

1. A process for the preparation of sitagliptin or its pharmaceutically acceptable salts of formula 1 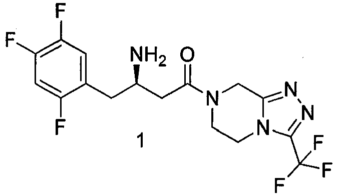 comprising the steps of
a) condensation of compound of formula 2 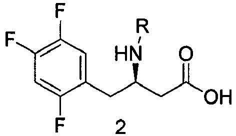 wherein R is amino protecting group which can be removed with compound of formula 3 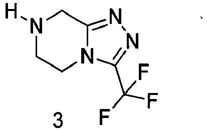 which may be used in its free base form or in a form of a salt in the presence of a coupling agent and a basic solvent that is *N*-methylimidazole to obtain the compound of formula 4 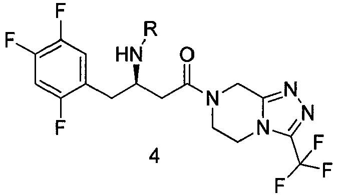 and
b) deprotection of compound of formula 4 to obtain sitagliptin base that can be further transformed into its pharmaceutically acceptable salt.

2. The process according to the claim 1 characterized that the amino protecting group which can be removed under acidic condition is selected from the group consisting of carbamate group, *tert*-Butyloxycarbonyl (BOC), Carbobenzyloxy (Cbz) group, *p-*Methoxybenzyl carbonyl (Moz or MeOZ) group, 9-Fluorenylmethyloxycarbonyl (FMOC) group, Acetyl (Ac) group, Benzoyl (Bz) group, Benzyl (Bn) group, *p*-Methoxybenzyl (PMB), 3,4-Dimethoxybenzyl (DMPM).

3. The process according to any previous claims characterized that the coupling agent is selected from the group consisting of phosgene, diphosgene, triphosgene, carbonildiimidazole, chlorothionyl imidazole, thionyl diimidazole, dicyclohexylcarbodiimide (DCC), of *N*-(3-Dimethylaminopropyl)-*N'*-ethylcarbodiimide hydrochloride-a (EDC-HCl), tetramethyluronium salts such as *O*-(benzotriazol-1-yl)-*N,N, N',N'*-tetramethyluronium hexafluorophosphate (HBTU), *O*-(benzotriazol-1-yl)-*N,N,N', N'*-tetramethyluronium tetrafluoroborate (TBTU), or *O*-(7-aza-benzotriazol-1-yl)-*N,N,N', N'*-tetramethyluronium hexafluorophosphate, (HATU).

4. The process according to any previous claims characterized that the HPLC purity of sitagliptin is from 99 to 100%.

5. The process according to any previous claims characterized that the percent enantiomeric excess of sitagliptin is more than 99.5% (by HPLC).

6. The process according to any previous claims characterized that sitagliptin is further converted into one of its pharmaceutically acceptable salts prepared from any inorganic or organic base or acid.

7. The process according to claim 2 **characterized in that** the amino protecting group which can be removed under acidic condition is carbamate group or BOC.

8. The process according to claim 3 **characterized in that** the coupling agent is N-(3-Dimethylaminopropyl)-*N*'-ethylcarbodiimide hydrochloride-a (EDC-HCl).

9. The process according to any one of the preceding claims **characterized in that** the process for the preparation of sitagliptin or its pharmaceutically acceptable salts of formula 1 is a one pot process for the preparation of sitagliptin or its pharmaceutically acceptable salts of formula 1.

10. The process according to any one of the preceding claims **characterized in that** in process step a) compound of formula 3 is used in the form of hydrochloride salt.

11. The process according to any one of the preceding claims **characterized in that** compound of formula 2 is a compound of formula 2 wherein R is an amino protecting group which can be removed under acidic condition.

12. The process according to any one of the preceding claims **characterized in that** sitagliptin is further converted into a pharmaceutically acceptable salt selected from the group consisting of hydrochloride, phosphate, dihydrogenphosphate, tartrate, benzensulfonate, sulfate, acetate.

13. Use of N-methylimidazole as an organic solvent in a condensation of a compound of formula 2 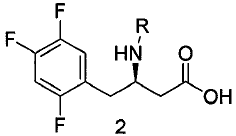 wherein R is an amino protecting group which can be removed with compound of formula 3 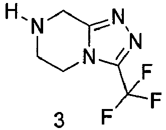 which may be used in its free base form or in a form of a salt to obtain the compound of formula 4

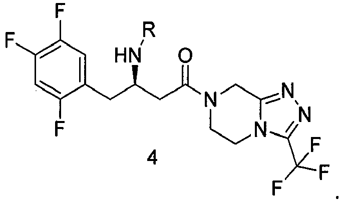

14. Use according to claim 13 wherein N-methylimidazole is also used as a solvent in the deprotection of a compound of formula 4 to obtain sitagliptin base.

## Patentansprüche

1. Verfahren zur Herstellung von Sitagliptin oder seinen pharmazeutisch verträglichen Salzen der Formel 1 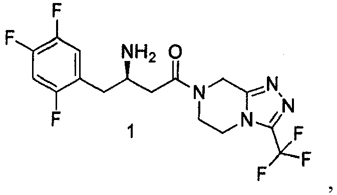 umfassend die Schritte von
a) Kondensation einer Verbindung der Formel 2 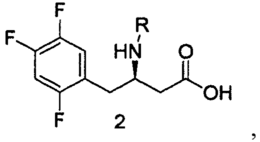 wobei R eine Aminoschutzgruppe ist, die mit der Verbindung der Formel 3 entfernt werden kann 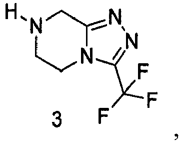 die in ihrer freien Basenform oder in einer Form eines Salzes in der Gegenwart eines Kupplungsmittels und eines basischen Lösungsmittels, welches *N*-Methylimidazol ist, verwendet werden kann, um die Verbindung der Formel 4 zu erhalten 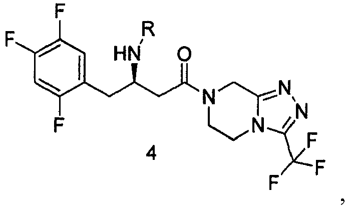 und
b) Entschützen einer Verbindung der Formel 4, um Sitagliptin-Base zu erhalten, die weiter in ihr pharmazeutisch verträgliches Salz umgewandelt werden kann.

2. Verfahren gemäß Anspruch 1, **dadurch gekennzeichnet, dass** die Aminoschutzgruppe, die unter saurer Bedingung entfernt werden kann, aus der Gruppe, bestehend aus Carbamatgruppe, *tert*-Butyloxycarbonyl (BOC), Carbobenzyloxy (Cbz)-Gruppe, *p*-Methoxybenzylcarbonyl (Moz oder MeOZ)-Gruppe, 9-Fluorenylmethyloxycarbonyl (FMOC)-Gruppe, Acetyl (Ac)-Gruppe, Benzoyl (Bz)-Gruppe, Benzyl (Bn)-Gruppe, *p*-Methoxybenzyl (PMB), 3,4-Dimethoxybenzyl (DMPM), ausgewählt ist.

3. Verfahren gemäß jedweden vorstehenden Ansprüchen, **dadurch gekennzeichnet, dass** das Kupplungsmittel aus der Gruppe, bestehend aus Phosgen, Diphosgen, Triphosgen, Carbonildiimidazol, Chlorthionylimidazol, Thionyldiimidazol, Dicyclohexylcarbodiimid (DCC), aus *N*-(3-Dimethylaminopropyl)-*N'-*ethylcarbodiimid-Hydrochlorid-a (EDC-HCl), Tetramethyluroniumsalzen wie *O*-(Benzotriazol-1-yl)-*N,N,N',N'*-tetramethyluroniumhexafluorphosphat (HBTU), *O*-(Benzotriazol-1-yl)-*N,N,N',N'*-tetramethyluroniumtetrafluorborat (TBTU) oder *O-*(7-Azabenzotriazol-1-yl)-*N,N,N'-N'*-tetramethyluroniumhexafluorphosphat (HATU), ausgewählt ist.

4. Verfahren gemäß jedweden vorstehenden Ansprüchen, **dadurch gekennzeichnet, dass** die HPLC-Reinheit von Sitagliptin 99 bis 100 % beträgt.

5. Verfahren gemäß jedweden vorstehenden Ansprüchen, **dadurch gekennzeichnet, dass** der prozentuale Enantiomerenüberschuss von Sitagliptin mehr als 99,5 % (nach HPLC) beträgt.

6. Verfahren gemäß jedweden vorstehenden Ansprüchen, **dadurch gekennzeichnet, dass** Sitagliptin weiter in eines seiner pharmazeutisch verträglichen Salze, hergestellt aus jedweder anorganischen oder organischen Base oder Säure, umgewandelt wird.

7. Verfahren gemäß Anspruch 2, **dadurch gekennzeichnet, dass** die Aminoschutzgruppe, die unter saurer Bedingung entfernt werden kann, Carbamatgruppe oder BOC ist.

8. Verfahren gemäß Anspruch 3, **dadurch gekennzeichnet, dass** das Kupplungsmittel *N*-(3-Dimethylaminopropyl)-*N'*-ethylcarbodiimid-Hydrochlorid-a (EDC-HCl) ist.

9. Verfahren gemäß einem der vorhergehenden Ansprüche, **dadurch gekennzeichnet, dass** das Verfahren zur Herstellung von Sitagliptin oder seinen pharmazeutisch verträglichen Salzen der Formel 1 ein Ein-Topf-Verfahren zur Herstellung von Sitagliptin oder seinen pharmazeutisch verträglichen Salzen der Formel 1 ist.

10. Verfahren gemäß einem der vorhergehenden Ansprüche, **dadurch gekennzeichnet, dass** im Verfahrensschritt a) die Verbindung der Formel 3 in der Form eines Hydrochlorid-Salzes verwendet wird.

11. Verfahren gemäß einem der vorhergehenden Ansprüche, **dadurch gekennzeichnet, dass** die Verbindung der Formel 2 eine Verbindung der Formel 2, wobei R eine Aminoschutzgruppe ist, die unter saurer Bedingung entfernt werden kann, ist.

12. Verfahren gemäß einem der vorhergehenden Ansprüche, **dadurch gekennzeichnet, dass** Sitagliptin weiter in ein pharmazeutisch verträgliches Salz, ausgewählt aus der Gruppe, bestehend aus Hydrochlorid, Phosphat, Dihydrogenphosphat, Tartrat, Benzensulfonat, Sulfat, Acetat, umgewandelt wird.

13. Verwendung von N-Methylimidazol als ein organisches Lösungsmittel in einer Kondensation einer Verbindung der Formel 2 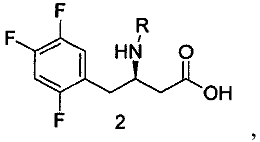 wobei R eine Aminoschutzgruppe ist, die mit der Verbindung der Formel 3 entfernt werden kann 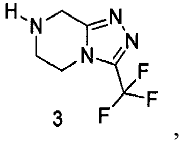 die in ihrer freien Basenform oder in einer Form eines Salzes verwendet werden kann, um die Verbindung der Formel 4 zu erhalten

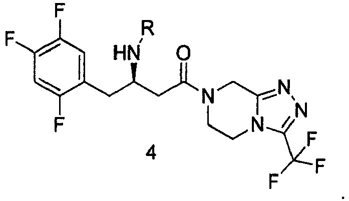

14. Verwendung gemäß Anspruch 13, wobei N-Methylimidazol auch als ein Lösungsmittel beim Entschützen einer Verbindung der Formel 4, um Sitagliptin-Base zu erhalten, verwendet wird.

## Revendications

1. Un procédé de préparation de sitagliptine ou de ses sels pharmaceutiquement acceptables de formule 1 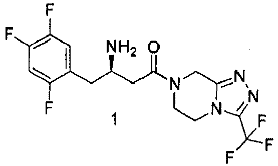 comprenant les étapes de
a) condensation du composé de formule 2 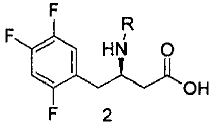 dans laquelle R est un groupe amino protecteur, qui peut être éliminé avec le composé de formule 3 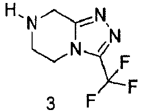 qui peut être utilisé sous sa forme de base libre ou sous la forme d'un sel, en présence d'un agent couplant et d'un solvant basique, qui est le N-méthylimidazole, pour obtenir le composé de formule 4 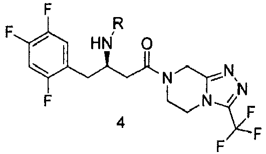 et
b) déprotection du composé du formule 4 pour obtenir la sitagliptine base, qui peut être transformée ultérieurement en son sel pharmaceutiquement acceptable.

2. Le procédé selon la revendication 1, **caractérisé en ce que** le groupe amino protecteur, qui peut être éliminé dans des conditions acides, est choisi parmi le groupe comprenant un groupe carbamate, *tert-*butyloxycarbonyle (BOC), un groupe carbobenzyloxy (Cbz), un groupe p-méthoxybenzyl carbonyle (Moz ou MeOZ), un groupe 9-fluorénylméthyloxycarbonyle (FMOC), un groupe acétyle (Ac), un groupe benzoyle (Bz), un groupe benzyle (Bn), un groupe p-méthoxybenzyle (PMB) et un groupe 3,4-diméthoxybenzyle (DMPM).

3. Le procédé selon l'une quelconque des revendications précédentes, **caractérisé en ce que** l'agent couplant est choisi parmi le groupe constitué du phosgène, diphosgène, triphosgène, carbonildiimidazole, chlorothionyl imidazole, thionyl diimidazole, dicyclohexylcarbodiimide (DCC), du chlorhydrate-a de *N*-(3-diméthylaminopropyl)-*N'*-éthylcarbodiimide (EDC-HCl), des sels de tétraméthyluronium tels que l'hexafluorophosphate d'*O*-(benzotriazol-1-yl)-*N*, *N,N',N'*-tétraméthyluronium (HBTU), le tétrafluoroborate d'*O*-(benzotriazol-1-yl)*-N,N,N',N'-*tétraméthyluronium (TBTU) ou l'hexafluorophosphate d'*O*-(7-aza-benzotriazol-1-yl)-*N,N,N',N'*-tétraméthyluronium, (HATU).

4. Le procédé selon l'une quelconque des revendications précédentes, **caractérisé en ce que** la pureté par HPLC de la sitagliptine est comprise entre 99 et 100 %.

5. Le procédé selon l'une quelconque des revendications précédentes, **caractérisé en ce que** le pourcentage d'excès énantiomérique de la sitagliptine est supérieur à 99,5 % (par HPLC).

6. Le procédé selon l'une quelconque des revendications précédentes, **caractérisé en ce que** la sitagliptine est convertie ultérieurement en l'un de ses sels pharmaceutiquement acceptables préparés à partir de n'importe quelle base ou acide inorganique ou organique.

7. Le procédé selon la revendication 2, **caractérisé en ce que** le groupe amino protecteur, qui peut être éliminé dans des conditions acides, est un groupe carbamate ou BOC.

8. Le procédé selon la revendication 3, **caractérisé en ce que** l'agent couplant est le chlorhydrate-a de *N*-(3-diméthylaminopropyl)-*N'-*éthylcarbodiimide (EDC-HCl).

9. Le procédé selon l'une quelconque des revendications précédentes, **caractérisé en ce que** le procédé de préparation de la sitagliptine ou de ses sels pharmaceutiquement acceptables de la formule 1 est un procédé en une seule cuve pour la préparation de sitagliptine ou de ses sels pharmaceutiquement acceptables de formule 1.

10. Le procédé selon l'une quelconque des revendications précédentes, **caractérisé en ce que** dans l'étape de procédé a), le composé de formule 3 est utilisé sous la forme de sel chlorhydrate.

11. Le procédé selon l'une quelconque des revendications précédentes, **caractérisé en ce que** le composé de formule 2 est un composé de formule 2 dans lequel R est un groupe amino protecteur, qui peut être éliminé dans des conditions acides.

12. Le procédé selon l'une quelconque des revendications précédentes, **caractérisé en ce que** la sitagliptine est convertie ultérieurement en un sel pharmaceutiquement acceptable choisi parmi le groupe comprenant le chlorhydrate, le phosphate, le dihydrogénophosphate, le tartrate, le benzènesulfonate, le sulfate et l'acétate.

13. Utilisation du N-méthylimidazole en tant que solvant organique dans une condensation d'un composé de formule 2 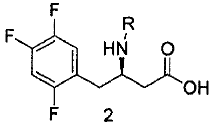 dans laquelle R est un groupe amino protecteur, qui peut être éliminé avec le composé de formule 3 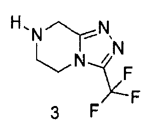 qui peut être utilisé sous sa forme de base libre ou sous la forme d'un sel, pour obtenir le composé de formule 4

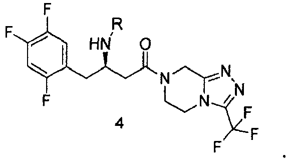

14. Utilisation selon la revendication 13, dans laquelle le N-méthylimidazole est également utilisé en tant que solvant dans la déprotection d'un composé de formule 4 pour obtenir la sitagliptine base.
